(19) Europäisches Patentamt
European Patent Office
Office européen des brevets

(11) Veröffentlichungsnummer : **0 114 984**
**B1**

(12) # EUROPÄISCHE PATENTSCHRIFT

(45) Veröffentlichungstag der Patentschrift : **21.10.87**

(51) Int. Cl.⁴ : **C 12 P 33/10**, C 12 P 33/16, C 07 J 1/00

(21) Anmeldenummer : **83112230.4**

(22) Anmeldetag : **06.12.83**

(54) **Verfahren zur Herstellung von 3,11-alpha-Dihydroxy-1,3,5(10)-östratrien-Derivaten.**

(30) Priorität : **23.12.82 DE 3248434**

(43) Veröffentlichungstag der Anmeldung : **08.08.84 Patentblatt 84/32**

(45) Bekanntmachung des Hinweises auf die Patenterteilung : **21.10.87 Patentblatt 87/43**

(84) Benannte Vertragsstaaten :
**AT BE CH DE FR GB IT LI LU NL SE**

(56) Entgegenhaltungen :
**EP-A- 0 028 309**
**DE-A- 1 909 152**
**US-A- 3 429 779**
**JOURNAL OF THE AMERICAN CHEMICAL SOCIETY, vo.88, nr.13, July 5, 1966, pages 3120-3128, WASHINGTON DC (US); L.L. SMITH et al.: "Microbiological Hydroxylation of Steroids of Unnatural Configuration"**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Petzoldt, Karl, Dr.**
**Flachsweg 10**
**D-1000 Berlin 38 (DE)**
Erfinder : **Neef, Günter, Dr.**
**Darstädter Strasse 9**
**D-1000 Berlin 15 (DE)**
Erfinder : **Eder, Ulrich, Dr.**
**Zeltinger Strasse 17**
**D-1000 Berlin 28 (DE)**

**EP 0 114 984 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von 3,11α-Dihydroxy-1,3,5(10)-östratrien-Derivaten der allgemeinen Formel I

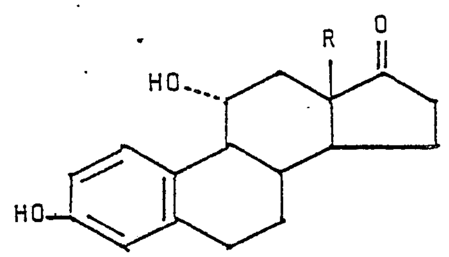

(I)

worin R einen Alkylrest mit maximal 4 Kohlenstoffatomen bedeutet, welches dadurch gekennzeichnet ist, daß man ein 3-Oxo-4-östren der allgemeinen Formel I

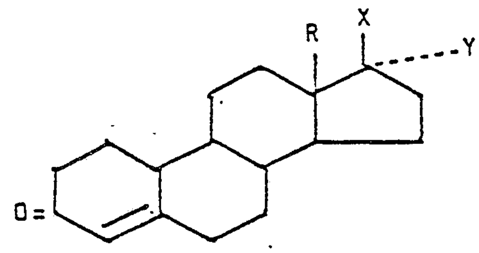

(II)

worin R die obengenannte Bedeutung besitzt und X und Y gemeinsam eine Oxogruppe darstellen oder X eine Hydroxygruppe und Y ein Wasserstoffatom darstellen, mit einer Kultur von Aspergillus ochraceus und nach erfolgter Hydroxylierung mit einer Kultur von Arthrobacter simplex oder Bacillus sphaericus fermentiert.

3,11α-Dihydroxy-1,3,5(10)-östratrien-Derivate der allgemeinen Formel I sind wichtige Zwischenprodukte zur Partialsynthese pharmakologisch wirksamer Steroide.

Es ist bekannt, daß Steroide mit aromatischem A-Ring als Substrate für mikrobiologische Umsetzungen wenig geeignet sind (K. Kieslich, Steroid Conversions, in R. H. Rose Microbiol. Bioconversions, Academic Press 1981, 425). Die an 3-Oxo-$\Delta^4$- oder 3-Hydroxy-$\Delta^5$-steroiden in hohen Ausbeuten durchführbare 11α-Hydroxylierung (W. Carney and H. L. Herzog, Microbial Transformations of Steroids, Academic Press 1967) ist auf Östron und Östradiol nicht übertragbar. Überraschenderweise erhält man mit Hilfe des erfindungsgemäßen Verfahrens in hohen Ausbeuten die 11α-Hydroxyverbindungen des Östradiols und seiner 18-Alkylhomologen. Dieses günstige Ergebnis war für den Fachmann nicht vorhersehbar, denn es war vorbekannt, daß man bei der Fermentation von 17β-Hydroxy-4-estren-3-on und 17β-Hydroxy-18-methyl-4-estren-3-on mit Aspergillus occhraceus Gemische Von 1β,10β und 11α-Hydroxyverbindungen dieser Substrate erhält (J. Amer. Chem. Soc., *88*, 1966, 3120-3128).

Der erste Reaktionsschritt des erfindungsgemäßen Verfahrens wird unter den Bedingungen durchgeführt, die man üblicherweise bei der mikrobiologischen Hydroxylierung von Steroiden mit Pilzkulturen anwendet. So werden zunächst in allgemein üblichen Vorversuchen die günstigsten Fermentationsbedingungen, wie zum Beispiel Auswahl des günstigsten Nährmediums, des geeigneten Substratlösungs- oder Suspensionsmittels, der Substratkonzentration, der technischen Bedingungen wie Temperatur, Belüftung, pH-Wert und der optimalen Zeiten für Germination, Substratzugabe und Substratkontakt am Enzym des Mikroorganismus analytisch, insbesondere dünnschichtchromatographisch, ermittelt.

Dabei hat sich gezeigt, daß es zweckmäßig ist, Konzentrationen von etwa 100-2 000 mg Substrat pro Liter Nährmedium einzusetzen. Der pH-Wert wird vorzugsweise auf einen Wert im Bereich von 5-7 eingestellt. Die Züchtungstemperatur liegt im Bereich von 20-40 °C, vorzugsweise von 25-35 °C. Zur Belüftung werden vorzugsweise 0,5 bis 5 Liter Luft pro Minute pro Liter Kulturbrühe zugeführt. Die Umwandlung des Substrates wird zweckmäßigerweise durch dünnschichtchromatographische Analyse von Probeextrakten verfolgt. Die Fermentationsdauer beträgt etwa 20 bis 80 Stunden.

Die für diesen ersten Reaktionsschritt benötigten Aspergillus ochraceus Stämme stehen der Fachwelt bei anerkannten Mikroorganismen-Sammlungen frei zur Verfügung. Geeignete Stämme sind beispielsweise : Aspergillus ochraceus CBS 13 252, ATCC 1008, ATCC 12 337, ATCC 18 500 oder NRRL 405.

Nach beendeter Hydroxylierung wird die erhaltene Hydroxyverbindung zweckmäßigerweise nicht isoliert, sondern der Fermentationsansatz mit einer 6 bis 24 Stunde alten Kultur von Arthrobacter simplex oder Bacillus sphaericus versetzt, welche unter den Bedingungen hergestellt wurde, die man üblicherweise zur Anzucht von Bakterienkulturen verwendet.

Die Züchtungstemperatur liegt im Bereich von 20-40 °C, vorzugsweise von 25-35 °C. Zur Belüftung werden vorzugsweise 0,5 bis 5 Liter Luft pro Minute pro Liter Kulturbrühe zugeführt.

Die für diesen zweiten Reaktionsschritt Arthrobacter simplex oder Bacillus sphaericus Stämme stehen ebenfalls bei anerkannten Mikroorganismen-Sammlungen frei zur Verfügung. Geeignete Stämme sind beispielsweise: Arthrobacter simplex IFO (3530), ATCC 13 260 und ATCC 6 946 oder Bacillus sphaericus ATCC 7 054, ATCC 7 055, ATCC 12 488 oder ATCC 13 805.

Der Verlauf des zweiten Reaktionschritts wird ebenfalls analytisch, beispielsweise dünnschichtchromatographisch, verfolgt. Die Fermentationsdauer dieses Schrittes beträgt etwa 40 bis 80 Stunden.

Nach erfolgter Fermentation werden die Fermentationsprodukte in an sich bekannter Weise isoliert. Die Isolierung kann zum Beispiel in der Weise erfolgen, daß man die Fermentationsansätze mit einem nicht mit Wasser mischbaren organischen Lösungsmittel wie Äthylacetat, Butylacetat oder Methylisobutylketon, extrahiert, die Extrakte einengt und die so erhaltenen Rohprodukte gegebenenfalls durch Chromatographie und/oder Kristallisation reinigt.

Bevorzugte Ausgangsverbindungen des erfindungsgemäßen Verfahrens sind solche der allgemeinen Formel II, welche als Substituenten R eine Methylgruppe oder eine Ethylgruppe tragen.

Die nachfolgenden Ausführungsbeispiele dienen zur Erläuterung des erfindungsgemäßen Verfahrens.

## Beispiel 1

Ein 2 Liter-Erlenmeyerkolben, der 500 ml einer 30 Minuten bei 120 °C im Autoklaven sterilisierten Nährlösung aus 1 % Corn steep liquor, 1,25 % Sojabohnenmehl und 0,005 % Sojaöl enthält, wird mit einer Schrägröhrchen-Kultur des Stammes Aspergillus ochraceus (ATCC 1008) beimpft und 2 1/2 Tage auf einem Rotationsschüttler geschüttelt.

Mit 250 ml dieser Anzuchtskultur wird ein 20 Liter-Vorfermenter beimpft, der mit 15 l eines 60 Minuten bei 121 °C und 1,1 bar Überdruck sterilisierten Mediums, bestehend aus 1 % Corn steep liquor, 1 % Sojabohnenmehl und 0,005 % Sojaöl, eingestellt auf pH 6,2, gefüllt ist. Unter Zugabe von Silicon SH als Antischaummittel wird bei 29 °C und 0,7 bar Überdruck unter Belüftung (15 l/Min.) und Rühren (220 U/Min.) 24 Stunden germiniert.

Danach werden 0,9 Liter dieser Kultur unter sterilen Bedingungen entnommen und damit ein 50 Liter-Hauptfermenter beimpft, der mit 15 Liter sterilisiertem Nährmedium der gleichen Zusammensetzung wie die Vorfermenterkultur beschickt ist. Nach einer Anwachsphase von 12 Stunden unter Vorfermenterbedingungen wird eine sterilfiltrierte Lösung von 10,5 g 19-Nortestosteron in 150 ml Dimethylformamid hinzugegeben und weiter gerührt und belüftet. Der Verlauf der Fermentation wird durch Entnahme von Proben kontrolliert, die mittels Methylisobutylketon extrahiert und dünnschichtchromatographisch analysiert werden. Nach einer Kontaktzeit von ca. 60 Stunden ist die Hydroxylierung vollständig.

Parallel zur Hydroxylierung werden in einem zweiten Fermenter 15 l flüssiges Nährmedium, bestehend aus 1 % Hefeextrakt, 0,5 % Corn steep liquor und 0,05 % Stärkezucker sterilisiert, mit 250 ml einer 2-tägigen Arthrobacter simplex — Kultur (ATCC 6946) beimpft und unter Belüftung (15 l/Min.) und Rühren (220 U/Min.) bei 30 °C germiniert. Der Zeitpunkt der Beimpfung ist dabei so zu wählen, daß die Kultur bei Beendigung der Hydroxylierung eine Anwachsphase von ca. 18-24 Stunden hinter sich hat. Sodann wird die gesamte Bakterien-Kultur in den halbgefüllten Substratfermenter überführt und mit der A. ochraceus — Kultur vereinigt. Die Bebrütung wird unter den oben genannten Bedingungen fortgesetzt und die nun einsetzende Aromatisierung weiterhin dünnschichtchromatographisch verfolgt.

Nach weiteren 60-70 Stunden Kontaktzeit ist auch die Aromatisierung beendet. Die Kulturbrühe wird nun mit der Hälfte ihres Volumens und anschließend noch zweimal mit 1/3 des Volumens an Methylisobutylketon extrahiert, die Extrakte vereinigt und im Vakuum zur Trockne eingedampft. Den Rückstand rührt man zur Entfernung des Antischaummittels 2 Stunden mit Hexan, wobei das Hexan mehrmals ersetzt wird und kristallisiert schließlich aus Aceton um.

Man erhält 8,2 g 3,11α-Dihydroxy-1,3,5(10)-östratrien-17-on vom Schmelzpunkt 267-269 °C.

## Beispiel 2

Unter den Bedingungen des Beispiels 1a werden zu einem 50 Liter-Fermenter, der 15 Liter einer Aspergillus ochraceus-Kultur enthält, 15 g 18-Methyl-19-nor-4-östren-3,17-dion, gelöst in 200 ml DMF, zugegeben und 36 Stunden incubiert. Anschließend wird der Ansatz mit 15 l einer 24 Stunden vorkultivierten Arthrobacter simplex — Kultur vereinigt und weitere 63 Stunden aerob fermentiert.

Zur Isolierung des Umwandlungsprodukts extrahiert man die Kulturbrühe mit Methylisobutylketon, dampft den Extrakt zur Trockne ein, entfernt das Antischaummittel durch Waschen mit Hexan und chromatographiert das Rohprodukt über eine kieselgelsäule (Elution mittels Dichlormethan + 12 % Aceton). Nach Kristallisation aus Methanol/Hexan erhält man 6,3 g 3,11α-Dihydroxy-18-methyl-1,3,5(10)-östratrien-17-on vom Schmelzpunkt 263-264 °C.

**Patentanspruch**

Verfahren zur Herstellung von 3,11α-Dihydroxy-1,3,5(10)-östratrien-Derivaten der allgemeinen Formel I

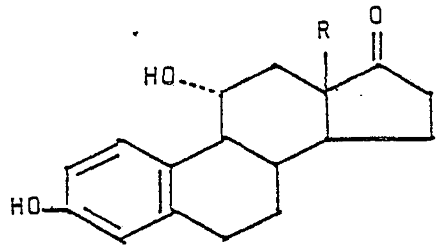

(I)

worin R einen Alkylrest mit maximal 4 Kohlenstoffatomen bedeutet, dadurch gekennzeichnet, daß man ein 3-Oxo-4-östren-Derivat der allgemeinen Formel II

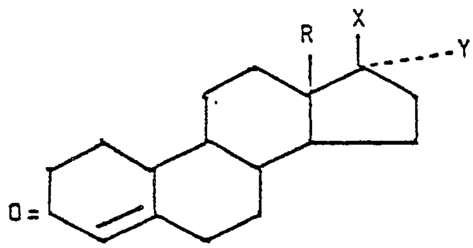

(II)

worin R die obengenannte Bedeutung besitzt und X und Y gemeinsam eine Oxogruppe darstellen oder X eine Hydroxygruppe und Y ein Wasserstoffatom darstellen, mit einer Kultur von Aspergillus ochraceus und nach erfolgter Hydroxylierung mit einer Kultur von Arthrobacter simplex oder Bacillus sphaericus fermentiert.

**Claim**

Process for the preparation of 3,11α-dihydroxy-1,3,5(10)-oestratriene derivatives of the general formula I

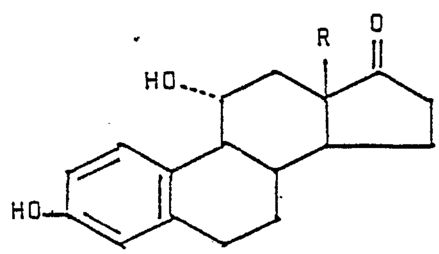

(I)

in which R represents an alkyl radical having a maximum of 4 carbon atoms, characterised in that a 3-oxo-4-oestrene derivative of the general formula II

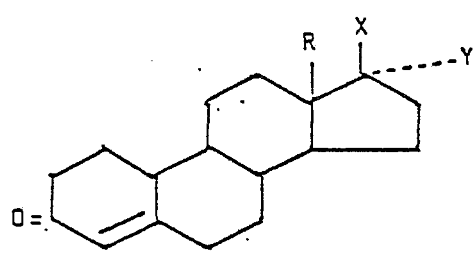

(II)

in which R has the meaning mentioned above and X and Y together represent an oxo group or X represents a hydroxy group and Y represents a hydrogen atom, is fermented with a culture of Aspergillus ochraceus and, when hydroxylation is complete, with a culture of Arthrobacter simplex or Bacillus sphaericus.

**Revendication**

Procédé de préparation de dérivés du dihydroxy-3,11α œstratriène-1,3,5(10) répondant à la formule générale I

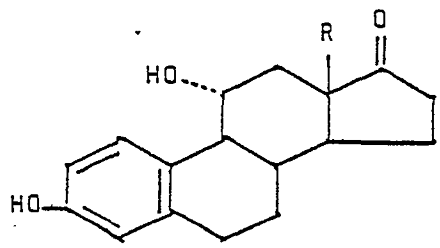

(I)

dans laquelle R représente un radical alkyle contenant au plus 4 atomes de carbone, procédé caractérisé en ce qu'on fait fermenter un oxo-3 œstrène-4 répondant à la formule générale II

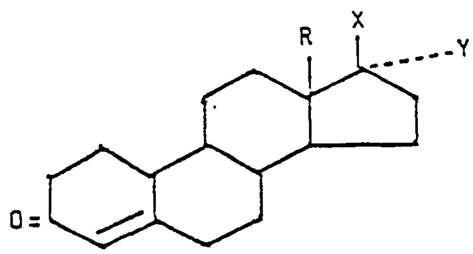

(II)

dans laquelle R a la signification précédemment donnée tandis que X et Y forment ensemble un radical oxo, ou encore X représente un radical hydroxy et Y un atome d'hydrogène, avec une culture d'Aspergillus ochraceus et, une fois l'hydroxylation effectuée, avec une culture d'Arthrobacter simplex ou de Bacillus sphaericus.